# EUROPEAN PATENT APPLICATION

(11) **EP 4 124 294 A1**
(43) Date of publication of application: **01.02.2023**
(21) Application number: 21188022.4
(22) Date of filing: 27.07.2021
(51) Int. Cl.: A61B 5/113, A61B 5/00

(54) **DEVICE AND METHOD USING RESPIRATORY INDUCTANCE PLETHYSMOGRAPHY**

(71) Applicant: Netsensing Technology Sàrl, 2000 Neuchâtel (CH)
(72) Inventor: Yazigi, Raja, 2520 La Neuveville (CH); Koller, Philippe, 2000 Neuchâtel (CH)
(74) Representative: P&TS SA (AG, Ltd.)

(57) **Abstract**

The present invention relates to a Respiratory Induction Plethysmography device comprising at least one folded wire extendable in a first direction (D1) and at least one second wire extendable in a second direction (D2), each of these wires being operated at different frequencies. The present invention further relates to disposable stretchable bands adapted therefor and to a method of RIP measurement, adapted for the identification of dorsal apnea.

## Description

### Technical domain

The present invention concerns a wearable device adapted for monitoring a patient during sleep. It relates in particular to a wearable device used for respiratory inductance plethysmography. It further relates to a method of monitoring the patient during sleep, and more particularly monitoring the sleep apnea syndrome.

### Related art

Sleep apnea syndrome (SAS) is a sleep disorder in which an individual's breathing repeatedly reduces during sleep, sometimes tens or hundreds of times. One common form of SAS is the obstructive sleep apnea (OSA) where the pause in breathing is due to a blocked airway, usually when the soft tissue in the back of the throat collapses during sleep. Another form of SAS is central sleep apnea (CSA) where the pause is commanded from the brain. Another form of the SAS is hypopnea where respiration is reduced along with a oxygen desaturation or a micro wake up.

If untreated, SAS can result in a number of health problems, including heart failure such as sudden cardiac death. Sudden death is widespread both among newborns and among adults.

The use of Respiratory Inductance Plethysmography (RIP) is commonly used to this end. Stretchable belts comprising an electrically conductive wire are placed around the chest and around the abdomen of a patient to monitor both thoracic and abdominal breath. The resonance frequency of the plethysmography wire loop varies during the breath of the patient since the area of the loop varies. The variation of the inductance of the wire loops related to the two belts can be analysed to determine the apnea events of the patient during the sleep.

The wires have been object of extensive alternatives to improve the sensitivity of the measurement. The document US2004225227A1 discloses an elastic belt wherein the conductor has hairpin-like feature. The document US6341504B1 provide devices comprising multiple parallel wires.

While the sensitivity has been a parameter of interest so far, several limitations remain with regard to the Respiratory Inductance Plethysmography. One of those resides in the fact that it can be difficult to distinguish the move related to the breath of the patient from the other movements that a patient can have during night. In addition, the known devices do not allow to determine the position of the patient. In particular, the known RIP belts only determine the area variation of the chest and abdomen of the patient without determining if the patient is lying on is back or on his face. It is thus necessary to use additional monitoring devices such as cameras or additional sensors such as 3D accelerometer, and to correlate the data of more than one device to determine if the apnea events occur when the patient his on his back or in another position. For example, the American Academy of Sleep Medicine (AASM) mentions that the dorsal apnea should be distinguish from the non-dorsal apnea. For example, the obstructive sleep apnea may be associated to a dorsal position of the patient, which may request specific treatment conditions.

Furthermore, the RIP belts currently used are disposable devices. They are used for a given patient, or for a predetermined period of monitoring. The RIP belts are designed to be combined with a non-disposable oscillators. They are thus certified to provide reliable measurements with such dedicated oscillators. In addition, no significant variation of measurement should occur from one RIP belt to another one.

### Short disclosure of the invention

An aim of the present invention is the provision of a wearable device adapted for Respiratory Inductance Plethysmography, that overcomes the shortcomings and limitations of the state of the art. It is in particular an aim to provide a wearable device which allows determining the dorsal position of a patient while detecting a sleep apnea event.

It is a further aim of the present invention to provide a disposable wearable device which is adapted to a given oscillator. It is also aimed at preventing usage of non-authorized or non-valid disposable device.

Another aim of the invention is to provide a method for monitoring a patient during sleep. In particular, the present method aims at detecting and/or identifying sleep apnea syndrome and related sleep apnea indications.

According to the invention, these aims are attained by the object of the independent claims, and further detailed in the claims dependant thereon.

With respect to what is known in the art, the invention provides the advantage of a concomitant determination of an apnea event and the position of a patient, in particular the dorsal position of a patient, using a single detection device. More particularly, the single detection device is a RIP device. It further provides the advantage of a reliable measurement, since only authorized disposable stretchable bands are used for the measurements. Other advantages will appear through the following disclosure.

### Short description of the drawings

Exemplar embodiments of the invention are disclosed in the description and illustrated by the drawings in which:
- Figure 1 : Schematic Representation of a device according to an embodiment of the present invention
- Figure 2: Schematic Representation of a device according to another embodiment of the present invention

### Examples of embodiments of the present invention

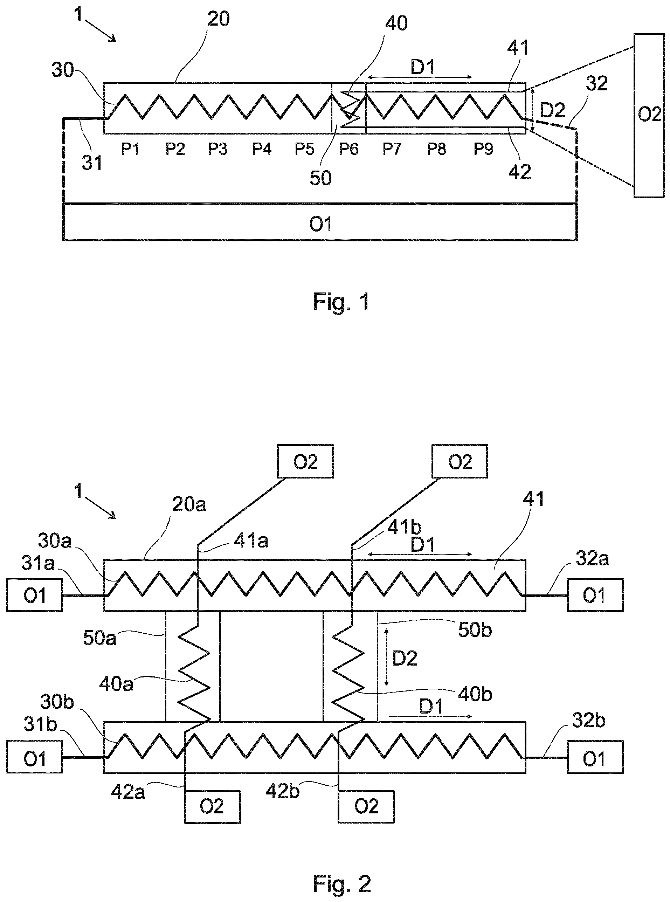

With reference to figure 1, the wearable device 1 comprises at least one stretchable band **20** the extremities of which can be removably joined. To this end, any mean of connexion of the two extremities may be used (not represented), such as scratch, snaps, boucle, hook, clamp and related means. The stretchable band **20** is stretchable along a first stretch direction **D1.** Preferably, the first stretch direction **D1** corresponds to the longitudinal dimension of the band **20,** meaning the direction between the two extremities of the band **20.** The stretchable band **20** can be stretchable in the first stretch direction **D1** on all its length. Alternatively, it can comprise stretchable portions in the first stretch direction **D1,** which alternate with non-stretchable portions.

The stretchable band **20** is combined with at least a first wire **30** which is electrically conductive, and which is oriented along the first stretch direction **D1.** The first wire **30** is combined with the stretchable band **20** so as to not be straight when the stretchable band **20** is at rest, meaning not stretched. The first wire **30** occupies a certain width of the stretchable band **20,** between one side and the other one, either in a regular way or in a non-regular way. For example, the stretchable band **20** can have a width comprised between around 5 to 20 cm, or around 8 to 15 cm, such as about 10 cm. The length of the stretchable band **20** is typically comprised between around 80 cm to around 180 cm so as to be arranged around the thorax of a patient. Adjusting means may be included to better fit the stretchable band **20** to the patient. The first wire **30** is connected to the stretchable band **20** at specific points so that it is free to extend when the stretchable band **20** is stretched, while remaining combined with the stretchable band **20.** The term "combined" means associated to, or linked to, or integrated with. The first wire **20** is thus folded along the first stretch direction **D1.** The first wire **30** can be regularly folded along the full length of the stretchable band **20.** Alternatively, it can be folded only at portions of the stretchable band **20,** in particular when the stretchable band **20** comprises stretchable and non-stretchable portions. In this case, the folded portions of the first wire **30** correspond to the stretchable portions of the band **20.**

The first **31** and second **32** ends of the first wire **30** can be connected to an oscillator (not represented) adapted to pulse an electrical courant through the wire **30.** Depending on the stretching status of the stretchable band **20,** the inductance related to the first wire **30** varies and can be measured to determine the respiratory parameters. Among the measured parameters, one can cite the respiratory rate, the volume corresponding to each breath, the minute ventilation, the peak inspiratory flow, the fractional inspiratory time, the work of breathing, the peak and the mean of inspiratory and expiratory flow.

The stretchable band **20** can comprise more than one first wire **30** arranged along the first stretch direction **D1.** In case several first wires 30 are included or combined to the first stretchable band **20,** they may be all identical or be different from each other, or arranged differently from each other. For example, they can be differently folded, or have different size, or comprise folded portion at different positions of the stretchable band **20,** or combine several of these distinctions.

The stretchable band **20** can further comprise one or several second stretchable portions **50** which are stretchable in a second stretch direction **D2.** Preferably, the second stretch direction **D2** is a direction orthogonal to the first stretch direction **D1.** The second stretch direction **D2** preferably corresponds to the direction of the vertebral column of the patient. Each of the second stretchable portions **50** comprises at least a second wire **40,** which is electrically conductive, and which is oriented along the second stretch direction **D2.**

Preferably, the stretchable band **20** is stretchable in the first stretch direction **D1** and in the second stretch direction **D2** along its full length. It comprises one or several second wires **40** oriented along the second stretch direction **D2.**

The second wire **40** comprises a first **41** and a second **42** ends, which can be connected to an oscillator (not represented) adapted to pulse an electrical courant through the wire **30.** The second wire **40** comprises at least a folded portion which can be extended when the stretchable band **20** is stretched along the second stretch direction **D2.** The folded portion may be limited to the width of the first stretchable band **20.** It can have for example a length of around 5 to 10 cm. The inductive loop thus comprises said folded portion and the non-folded conductive portions of the second wire, wherein at least the folded portion, or only the folded portion, is oriented along the second stretch direction **D2.**

According to an embodiment, the first wire **30** and the second wire **40** are both combined to the same stretchable band **20.** In this case, the stretchable band **20** is stretchable, at least partly in both the first stretch direction **D1** and the second stretch direction **D2.** Alternatively, the first wire **30** is combined to the stretchable band **20** and the second wire **40** is combined to a different stretchable band, stretchable in the second stretch direction D2 and crossing the first stretchable band **20.** In this last case, the length of the folded portion of the second wire **40** can be larger than the width of the first stretchable band **20.**

The second wire **40,** whether combined to the same stretchable band **20** as the first wire **30** or to a different one, may cross the first wire **30** at a predetermine position **P** along the length of the stretchable band **20.** For example, the second wire **40** may cross the first wire **30** at a position close to one of the extremities of the stretchable band **20.** Alternatively, it can cross the first wire **30** at a more central position **P** of the stretchable band **20.** Such a predetermined position **P** can correspond to a portion of the body of the patient once the wearable device is placed on the patient. For example, a position **P** close to an extremity of the stretchable band **20** can correspond to a frontal part of the patient such as the chest or the abdomen, when the stretchable band **20** is locked on a frontal side of the patient. A more central position **P,** on the stretchable band **20,** would thus correspond to a dorsal part of the patient. Other position **P** corresponding to a lateral part of the patient may also be predetermined so that a wire **40** can be arranged thereon.

The stretchable band **20** can comprise only one predetermined position **P,** wherein a second wire **40** is arranged, crossing the first wire **30.** The predetermined position **P** can be arranged so that the second wire **40** is placed on a dorsal or a facial or a lateral position of the patient, once the wearable device is placed. Alternatively, the stretchable band **20** can comprise at least two predetermined positions **P** where a second wire **40** crosse the first wire **30,** each of the predetermined position **P** corresponding to a dorsal or a facial or a lateral portion of the patient's body. For example, two predetermined position P may be arranged, wherein one corresponds to a dorsal part of the patient and the other one corresponds to the facial part of the patient. The overall arrangement may of course be easily adapted according to the needs.

According to an advantageous arrangement, the first wire **30** is combined to the stretchable band **20** as above described, and the second wire **40** is combined to a different stretchable band, crossing the first stretchable band **20.** The second stretchable band, comprising the second wire **40** can thus cross the first stretchable band **20** at any position **P,** selected according to the needs. The second stretchable band can for example be placed at a position corresponding to the dorsal part or to the facial part of the patient. Several second bands can also be envisaged and freely placed at the desired positions **P.** The second wire **40** comprises at least one folded portion oriented along the second stretch direction **D2** so that an elongation of the second stretchable band can be detected. The length of such a folded portion can be comprised between around 5 to 10 cm, which can be sufficient to detect an elongation of the second stretchable band. It can also be longer if necessary.

Although the first stretchable band **20** may be independent from one or several second stretchable bands, crossing it, attachment points may be arranged on the first stretchable band **20,** and/or on the second crossing stretchable bands. Such attachment points can be of any suitable type such as snaps, snap button, hook, passer-by etc. The attachment points may be localised on predetermined positions **P1, P2, P3...P9** along the first stretchable band **20,** so that the second stretchable bands can be arranged across the first stretching band **20** at predetermined and reproducible positions.

According to an embodiment, the folded length of the second wire **40** is fully contained in the width of the first stretchable band **20.** This is for example the case when both the first **30** and second **40** wires are combined to the same first stretchable band 20. According to another embodiment, the second stretchable band, comprising the second wire **40,** can form a loop around the patient's body, passing above his shoulders like suspenders.

The wearable device 1 may thus comprise at least one first stretchable band **20** comprising a first wire **30** and at least one second wire **40** crossing the first wire **30,** the second wire being combined with the first stretchable band **20** or with another stretchable band. Preferably, the wearable device **1** comprises a set of one or several first stretchable bands **20,** each comprising at least one first wire **30** comprising folded portions arranged along the first stretch direction **D1,** and one or several second wire **40,** being combined to said first stretchable band **20** or to independent one, and comprising at least one folded portion arranged along the second stretch direction **D2.**

Figure 2 illustrates an alternative arrangement wherein two first stretchable bands **20a, 20b** are arranged parallel to one another, each one comprising at least one first wire **30a, 30b,** said arrangement further comprising one or more second stretchable bands **50a, 50b,** each one comprising at least one second wire **40a, 40b.** The first stretchable bands **20a, 20b** are stretchable in the first stretch direction **D1** and the second stretchable bands **50a, 50b** are stretchable in the second stretch direction **D2.** The one or several second stretchable bands **50a, 50b** can be non-removably linked to the two first stretchable bands **20a, 20b.** Alternatively, the one or several second stretchable bands **50a, 50b** can be removably linked to the two first stretchable bands **20a, 20b** by mean of some attachment points such as snaps, snap button, hook or equivalent means. The extremities of each second stretchable band **50a, 50b,** or some of them, can join the two parallel first stretchable bands **20a, 20b.** Alternatively, the second stretchable bands **50a, 50b,** or some of them, can cross one or both of the first stretchable band **20a, 20b.** The second stretchable bands **50a, 50b** can be arranged at predetermined positions corresponding to dorsal, a facial or a lateral position of the patient. Each of the second stretchable bands **50a, 50b** is combined to at least one second wire, having at least one folded portion oriented along the second stretch direction **D2.** The folded portion of the second wires **40a, 40b** may extend through the space between the two parallel first stretchable bands **20.** In this specific arrangement, the first stretchable band **20** may be stretchable in both **D1** and **D2** directions or only along the first stretch direction **D1.**

Independently of the special arrangement of the stretchable bands, both ends of each of the wires are connected to an oscillator. Preferably, the wearable device 1 comprises or is connected to two different oscillators. The first wire or wires of the first stretchable band **20,** or the first multiple stretchable bands **20a, 20b** when applicable, are connected to a first oscillator **O1.** The second wire or wires **40** are connected to a second oscillator **O2.** This is applicable when the second wire is combine to the same stretchable band as the first wire or to different stretchable bands. To this end, the wires comprise the necessary connexion means adapted to be electrically connected to the related oscillator. Preferably, the first oscillator **O1** works at a predetermine first frequency **F1** and the second oscillator **O2** works at a predetermine second frequency **F2.** Although the first **F1** and the second **F2** frequencies can be identical, they are preferably different from each other. For example, the frequencies **F2** related to the second wires **40** may be 2, or 5 or 10 or 20 fold higher than the frequencies related to the first wires **30.** The frequencies related to the second wires **40** may alternatively be lower than those related to the first wires **30,** by the same ratios. Other ratios may be envisaged according to the needs. The range of applicable frequencies is suitable for the RIP measurements. It can be for example comprised between few Hz, such as around 10 Hz, and few tenth of KHz such as around 10KHz or around 50KHz or more.

When the wearable device **1** comprises two parallel first stretchable bands **20a, 20b,** one of these two bands surrounds the chest of the patient and the other one surrounds the abdomen of the patient. The distance between the two stretchable bands **20a, 20b** can be predetermined to this extend. Alternatively, the distance between the two parallel first stretchable bands **20a, 20b** can be adapted. For example, when one or more second stretchable bands **50a, 50b** are arranged in between, their length can be object of an adjustment so as to optimise the relative position of the two parallel first stretchable band **20a, 20b.**

Whether the second wire **40** is comprised to the same first stretchable band **20** than the first wire or in an independent stretchable band, it is preferably arranged at a position corresponding the back of the patient.

The device here describe also comprises a central unit (not represented) adapted to measure the inductance and/or the variation of inductance. The first stretchable band **20** or assembly of first stretchable bands **20a, 20b,** can be connected to, or comprise, a first central unit adapted to determine the inductance or the variation of inductance related to the first wires **30.** The second stretchable band or assembly of second stretchable bands **50a, 50b,** can be connected to, or comprise, a second central unit adapted to determine the inductance or the variation of inductance related to the second wires **40.** The first central unit and the second central unit may be arranged as a single device or as two distinct devices as requested by the needs.

Although stretchable bands are here described, the device 1 also comprises garment or textiles or any related clothes comprising such stretchable bands, or being stretchable in such extend to correspond to the present disclosure.

The present disclosure also relates to a method for identifying sleep apnea syndrome using respiratory inductance plethysmography. It relates in particular to a method for determining the dorsal sleep apnea, using a combination of at least a first folded wire **30** and at least a second folded wire **40** which are stretchable in two different stretch directions **D1** and **D2.** The first wire or wires arranges so that they can extent around the chest and/or the abdomen of the patient. The inductance variation of the first wire or the first wires allow to monitor the breath of the patient. The second wire or the second wires **40** are arranged to cover the back side and/or the front side of the patient so as to identify extension on one of the back side or front side of the patient. The inductance variation of the second wire or second wires allow to determine the position of the patient. For example, when inductance varies on the second wires arranged on the facial side of the patient, it can be determined that the patient is sleeping on his back. On the contrary, when an inductance varies on the wire related to the back side of the patient, one can deduce that the patient sleeps on his facial side.

The second wire or second wires **40** may comprise a folded portion and a non-folded portion, so as to better localize the variation of the inductance. For example, a folded portion of a second wire **40** may be arranged either on the back side or on the facial side, and the non-folded portion may be arranged on the opposite side. By this way, a dorsal apnea can be identified. For example, the sensed data related to the first wire or to the firs wires **30** can be combined to the sensed data related to the second wire or wires **40** so that the position of the patient is determined when an apnea event is detected. According to another possible arrangement, a given second stretchable band may comprises several different second wires **40,** each comprising a folded portion, wherein the folded portion of one of these is arranged on a dorsal position and the folded portion of another wire is arranged on a facial position.

The method of the present disclosure comprises for instance a step a) of monitoring the breath of a patient by respiratory inductance plethysmography, using at least one first stretchable band **20, 20a, 20b** and a first oscillator **O1** working at a first frequency **F1.** The first stretchable band comprises at least one folded wire **30** and is arranged in the first stretch direction **D1.**

The method comprises a step b) of determining the position of the patient by respiratory inductance plethysmography, using at least a second electrically conductive wire **40,** arranged in a second stretch direction **D2** crossing the first direction **D1.** In particular, step b) allows to determine if the patient is sleeping on his back or not. Alternatively, step b) allows to determine whether the patient is sleeping on his back or on his facial side, or one of his lateral side.

The method further comprises a step c) of computing the data related to the first wire or wires **30** and the data related to the second wire or wires 40 for a given period of time so as to determine for an identified apnea event if the patient is sleeping on his back or not, or whether for an identified apnea event whether the patient is sleeping on his back, or on his facial side or on one of his lateral sides.

The method comprises a step d) of identifying a dorsal apnea, based on the previous steps. Such dorsal apnea is revealed when the patient is considered as sleeping on his back at a moment an apnea event or a succession of apnea events is detected.

The method may comprise a further step of calibrating the first wire or wires **30** and/or the second wire or wires **40.** The method also comprises a step of recognizing that the stretchable band **20** or the assembly of stretchable bands involved in the RIP measurement corresponds to reliable and authorized devices. A dedicated double measurement can be initiated to determine if the stretchable band **20** or the assembly of stretchable bands comprises at least one first wire **30** and at least on second wire **40,** and if the first wire comprises a folded portion extendable along the first stretch direction **D1** and the at least one second wire comprises a folded portion extendable along the second stretch direct **D2.** In case of absence of one or both of these first **30** and second **40** wires, or in case of damage thereof, the present device can provide an alert and/or stop or prevent the measurement. Additional control elements may be included to one or more of the above described stretchable bands. For example, **RFID** tag or equivalent means can be envisaged to follow the usage and/or the quality of the stretchable bands.

The present disclosure further relates to a disposable stretchable band **20** or an assembly of several disposable stretchable bands **20a, 20b, 50a, 50b** arranged as above described. Such a disposable stretchable band **20** or assembly of several disposable stretchable bands **20a, 20b, 50a, 50b** are preferably provided with two distinct electrical connexion means, one being adapted to connect a first wire **30** or a first assembly of wires **30** to a first oscillator **O1** and a second one adapted to connect a second wire **40** or a second assembly of wires **40** to a second oscillator **O2.**

## Claims

1. A wearable device (1) adapted to be worn by a patient, comprising at least one first stretchable band (20, 20a, 20b) comprising at least one folded first electrically conductive wire (30, 30a, 30b) having a first end (31, 31a, 31b) and a second end (32, 32a, 32b), both of its first end (31, 31a, 31b) and second end (32, 32a, 32b) being connected to a first oscillator (O1) working at a first frequency (F1), wherein said at least one first stretchable band (20) is stretchable in a first stretch direction (D1), **characterized in that** said wearable device (1) further comprises at least a second folded electrically conductive wire (40, 40a, 40b) having a first end (41, 41a, 41b) and a second end (42, 42a, 42b), both of its first end (41, 41a, 41b) and second end (42, 42a, 42b) being connected to a second oscillator (O2) working at a second frequency (F2), wherein said second folded wire (40, 40a, 40b) is adapted to be extended in a second stretch direction (D2) different from the first stretch direction (D1).

2. Wearable device according to claim 1, wherein said first (F1) and second (F2) frequencies are different from each other.

3. Wearable device according to one of claims 1 and 2, wherein both extremities of said at least one stretchable band (20, 20a, 20b) can be joined together by means of a suitable connexion mean.

4. Wearable device 1 according to one of claims 1 to 3, wherein said at least one first stretchable band (20, 20a, 20b) comprises predetermined positions (P1, P2, ...P9) along its length, and wherein said at least second conductive wire (40, 40a, 40b) can be placed across of said predetermined positions.

5. Wearable device according to claim 4, wherein at least one of said predetermined positions (P1, P2, ...P9) corresponds to a dorsal position of the patient.

6. Wearable device according to one of claims 1 to 5, wherein said at least one second wire (40), or some of said at least one second wire (40), are independent from said first stretchable band (20) and combined to at least one second stretchable band (50a, 50b) stretchable in said second stretch direction (D2).

7. Wearable device according to claim 6, wherein one or both of said first stretchable band (20) and said at least one second stretchable band (50a, 50b) comprise connexion means so as to maintain said first and second stretchable bands at predetermined relative positions.

8. Wearable device according to one of claims 1 to7, comprising two first stretchable bands (20a, 20b) arranged parallel to each other and one or more second stretchable bands (50a, 50b) being arranged orthogonally to said first stretchable band, wherein at least on of said second stretchable bands is arranged with regards to said first stretchable bands at a position corresponding to the dorsal side of the patient.

9. Wearable device according to one of claims 1 to 5, wherein said at least one second wire (40), or some of said at least one second wire (40), are combined to said first stretchable band (20) so as to cross the at least one first wire (30).

10. Wearable device according to claim 9, wherein said at least second wire comprises a folded portion extending within the width of said first stretchable band (20).

11. A disposable stretchable band or an assembly of disposable stretchable bands comprising at least one folded electrically conductive wire (30) adapted to be extended in a first stretch direction (D1) and at least one second folded electrically conductive wire (40) adapted to be extended in a second stretch direction (D2), wherein the ends of the at least one first wire (30) comprise electrical connexion means adapted to be connected to a first oscillator (O1) and wherein the ends of the second wire (40) comprise electrical connexion means adapted to be connected to a second oscillator (O2).

12. Disposable stretchable band or assembly of disposable stretchable bands according to claim 11, wherein said first oscillator (O1) works at a first frequency (F1) and wherein the second oscillator (O2) works at a second frequency (F2).

13. A method of monitoring the breath of a patient so as to identify dorsal apnea, comprising :
- a step a) of monitoring the breath of a patient by respiratory inductance plethysmography, using at least one first stretchable band (20, 20a, 20b) combined to at least one first folded wire (30) and a first oscillator (O1) working at a first frequency (F1), said first stretchable band being stretched in a first stretch direction (D1);
- a step b) of determining the position of the patient by respiratory inductance plethysmography, using at least a second folded electrically conductive wire (40), arranged in a second stretch direction (D2) crossing the first direction (D1);
- a step c) of computing the data related to said at least one first wire (30) and the data related to said at least one second wire (40) for a given period of time, and
- a step d) of identifying a dorsal apnea, based on the previous steps.

14. Method according to claim 13, wherein said second step b) of determining the position of the patient involves a second oscillator (O2) working at a second frequency (F2), different from said first frequency (F1).

15. Method according to one of claims 13 and 14, further comprising a step of checking the presence and the integrity of both the at least one first wire (30) and the at least one second wire (40).
